(19) Europäisches Patentamt
European Patent Office
Office européen des brevets

(11) Veröffentlichungsnummer: **0 240 659 B2**

(12) # NEUE EUROPÄISCHE PATENTSCHRIFT

(45) Veröffentlichungstag der neue Patentschrift: **26.10.94**

(51) Int. Cl.⁵: **C07D 209/48**, C07C 205/07, C07C 205/44, A01N 43/36, C07C 255/41, C07C 229/36, C07C 233/05

(21) Anmeldenummer: **87101378.5**

(22) Anmeldetag: **02.02.87**

Die Akte enthält technische Angaben, die nach dem Eingang der Anmeldung eingereicht wurden und die nicht in dieser Patentschrift enthalten sind.

(54) **N-substituierte 3,4,5,6-Tetrahydrophthalimide und deren Vorprodukte.**

(30) Priorität: **07.02.86 DE 3603789**

(43) Veröffentlichungstag der Anmeldung: **14.10.87 Patentblatt 87/42**

(45) Bekanntmachung des Hinweises auf die Patenterteilung: **20.03.91 Patentblatt 91/12**

(45) Bekanntmachung des Hinweises auf die Entsheidung über den Einspruch: **26.10.94 Patentblatt 94/43**

(84) Benannte Vertragsstaaten: **AT BE CH DE ES FR GB GR IT LI NL SE**

(56) Entgegenhaltungen:
**EP-A- 0 068 822**
**DE-A- 2 210 633**
**DE-A- 2 210 672**

**PATENT ABSTRACTS OF JAPAN, Band 9, Nr. 1 (C-259) [1724] 5. Januar 1985; & JP-A-59 155 358**

**Organic Reactions Bd. XV 1976. Wiley & Sons.**

(73) Patentinhaber: **BASF Aktiengesellschaft**
**Carl-Bosch-Strasse 38**
**D-67063 Ludwigshafen (DE)**

(72) Erfinder: **Plath, Peter, Dr.**
**Hans-Balcke-Strasse 13**
**D-6710 Frankenthal (DE)**
Erfinder: **Eicken, Karl, Dr.**
**Am Hüttenwingert 12**
**D-6707 Wachenheim (DE)**
Erfinder: **Goetz, Norbert, Dr.**
**Schoefferstrasse 25**
**D-6520 Worms (DE)**
Erfinder: **Wild, Jochen, Dr.**
**An der Marlach 7**
**D-6725 Deidesheim (DE)**
Erfinder: **Meyer, Norbert, Dr.**
**Dossenheimer Weg 22**
**D-6802 Ladenburg (DE)**
Erfinder: **Würzer, Bruno, Dr.**
**Rüdigerstrasse 13**
**D-6701 Otterstadt (DE)**

EP 0 240 659 B2

**Krajniak et al.Australian Journal of Chemistry vol.26 pp. 1337-1351. 1973**

## Beschreibung

Die vorliegende Erfindung betrifft neue N-substituierte 3,4,5,6-Tetrahydrophthalimide und deren Vorprodukte der allgemeinen Formel I

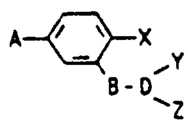

(I)

in der die Substituenten folgende Bedeutung haben:

A $-NO_2$, $-NH_2$ oder

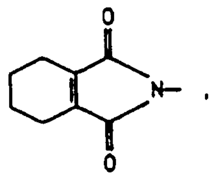

,

entsprechend den Verbindungen Ia, Ib und Ic.

B $-CH_2-$, $-CH_2-CHR^1-$, $-CH_2-CHR^1-CH_2-$, $-CH=$, $-CH=CR^1-$ oder $-CH=CR^1-CH=$, wobei der Rest $R^1$ für -H, -Cl, -Br oder $-CH_3$ steht,

D je nach der Endgruppe von B

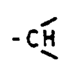 oder 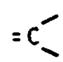 .

X -H, -Cl oder -Br,

Y -H, $C_1$-$C_7$-Alkyl, -Cl, -Br, -CN, $CONH_2$ oder $-CO_2R^2$, worin der Rest $R^2$ für H, $C_1$-$C_6$-Alkyl, $C_1$-$C_4$-Alkoxy-$C_2$-$C_4$-alkyl, $C_1$-$C_4$-Alkylmercapto-$C_2$-$C_4$-alkyl, Propargyl, Benzyl, durch F oder Cl bis zu dreifach substituiertes $C_2$-$C_4$-Alkyl oder durch $-CH_3$ oder -Cl substituiertes Allyl steht

Z

$-COOR^2$, $-CONR^3R^4$,

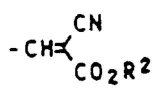

oder $-COR^2$,

worin die Reste $R^3$ und $R^4$ H und $C_1$-$C_4$-Alkyl bedeuten oder gemeinsam einen cycloaliphatischen 5- oder 6-Ring bilden, dessen Kohlenstoffkette durch ein Sauerstoffatom unterbrochen sein kann.

Außerdem betrifft die Erfindung die Herstellung der Verbindungen der allgemeinen Formel I, die Verwendung der N-substituierten 3,4,5,6-Tetrahydrophthalimide Ic als Herbizide, sowie Herbizide, welche die Verbindungen der Formel Ic enthalten.

Aus der JP-OS-59/155 358 sind N-substituierte 3,4,5,6-Tetrahydrophthalimide des Typs I' bekannt,

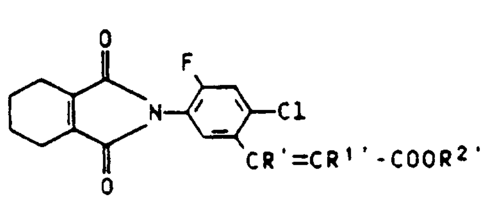

(I')

in denen R' und $R^{1'}$ Wasserstoff oder Methyl und $R^{2'}$ eine Alkylgruppe, besonders Ethyl, bedeuten.

Ähnliche Verbindungen, z.B. I''

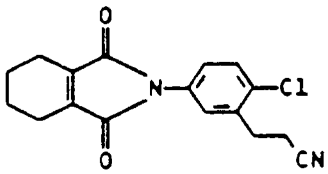

(I'')

sind aus der EP-OS-68 822 bekannt.

Diese Verbindungen wurden als Herbizide empfohlen, lassen jedoch in dieser Eigenschaft zu wünschen übrig.

Der Erfindung lag daher die Aufgabe zurgrunde, wirksamere Herbizide bereitzustellen.

Demgemäß wurden die eingangs definierten neuen N-substituierten 3,4,5,6-Tetrahydrophthalimide Ic sowie deren Vorprodukte Ia und Ib gefunden. Weiterhin wurde gefunden, daß die Verbindungen Ic sich hervorragend als Herbizide eignen.

Außerdem wurden Verfahren zur Herstellung der Verbindungen Ia-Ic gemaß der Patentansprüche 4 bis 8 gefunden.

Charakteristisch für die allgemeine herbizide Wirkung von Ic ist die 3,4,5,6-Tetrahydrophthalimidgruppe.

Im Hinblick auf die Wirkungsverstärkung und die selektive Wirkung der Herbizide übt dagegen der Molekülteil

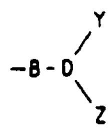

einen günstigen Einfluß aus.

Ebenfalls als herbizid wirksam wurden N-Phenyl-3,4,5,6-tetrahydrophthal-säureimide beschrieben, die in 2- und 4-Position des Phenylrings jeweils Wasserstoff oder ein Halogenatom tragen können und die zusätzlich in 5-Position des Phenylrings einen Acylrest, einen Acylketalrest oder einen Acyloximetherrest tragen können (EP-A 207 894).

Im einzelnen sind die Verbindungen des Typs I nach folgenden Methoden erhältlich:

a) Herstellung der Nitrobenzolderivate Ia

Man geht von 2-Halogen-5-nitrobenzaldehyden IIa

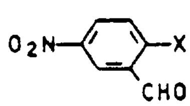

(IIa)

oder von 2-Halogen-5-nitrophenylacetaldehyden $IIa^{I}$

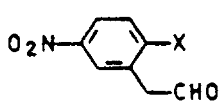

(IIa')

oder von 2-Halogen-5-nitrozimtaldehyden $IIa^{II}$

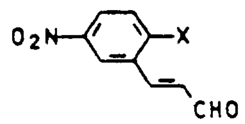

(IIa'')

aus und setzt diese in an sich bekannter Weise mit Cyanacetamiden, Cyanessigsäureestern, Malonsäurediestern, Malonsäuremonoestern oder mit $C_3$-$C_5$-Aldehyden um. Bei Verwendung von Malonsäuremonoestern wird das Decarboxylierungsprodukt $IIa^{III}$ erhalten.

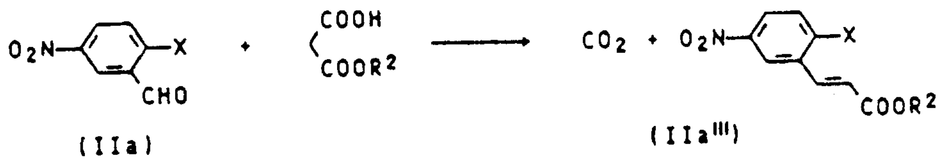

(IIa) (IIa''')

$\alpha$-($C_1$-$C_3$)-Alkylzimtsäuren des Typs $IIa^{IV}$ und ihre Derivate sind durch Umsetzung mit $C_3$-$C_5$-Aldehyden unter basischen Bedingungen und anschließender Oxidation leicht zugänglich.

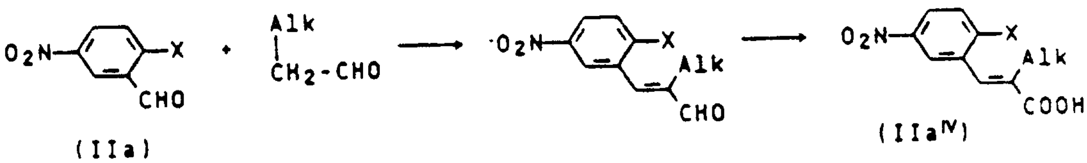

(IIa) ($IIa^{IV}$)

Die Aufarbeitung erfolgt wie üblich. Die Reinigung von Ia kann, falls überhaupt erforderlich, durch Umkristallisation vorgenommen werden, wozu sich meistens Alkohol/Wasser-Gemische eignen. Die Nitrobenzaldehyde IIa und Nitrozimtaldehyde IIa" sind bekannt oder nach bekannten Methoden erhältlich, so daß sich nähere Ausführungen hierzu erübrigen.

b) Herstellung der Anilinderivate Ib

Durch katalytische Hydrierung der Nitrobenzolderivate Ia entstehen die in der Seitenkette B-D gesättigten Anilinderivate. Selektive katalytische Hydrierung z.B. mit Patinoxid als Katalysator (Rylander; Catalytic Hydrogenation over Platinum Metals, Seite 176, Academic Press, New York 1967), sowie die Verwendung von Metallen, wie Eisen, zur Reduktion in an sich bekannter Weise lassen die in der Seitenkette vorhandenen Doppelbindungen unangetastet.

c) Herstellung der N-substituierten 3,4,5,6-Tetrahydrophthalimide Ic

Hierzu setzt man das 3,4,5,6-Tetrahydrophthalsäureanhydrid mit einem Anilin des Typs Ib nach an sich bekannten Methoden um. Die Umsetzung erfolgte beispielsweise in siedenden Carbonsäuren wie z.B. Essigsäure, Propionsäure oder Buttersäure. Eine andere Möglichkeit besteht in der Reaktion in einem inerten Lösungsmittel unter kontinuierlichem Entfernen des Reaktionswassers in Gegenwart von katalytischen Mengen einer Säure, vorzugsweise p-Toluolsulfonsäure. Als inerte Lösungsmittel eignen sich beispielsweise Benzolderivate, wie Toluol, Benzol, Chlorbenzol oder Xylol.

Das Halogen X des Aromaten läßt sich auch am Ende der Reaktionssequenz in Ic durch Umsetzung mit einem Halogenierungsmittel, z.B. Sulfurylchlorid in organischen Säuren bei 80 bis 130 °C einführen.

Chlor- und bromhaltige Gruppierungen B-D lassen sich in der Weise herstellen, daß man von ungesättigten Gruppierungen B-D ausgeht, diese zu den Dihalogenverbindungen umsetzt und hieraus gegebenenfalls durch Dehydrohalogenierung zum Monohalogenid gelangt.

Unter den Verbindungen Ic sind diejenigen bevorzugt, in denen die Gruppierung B $-CH_2-$ und $-CH=$ bedeutet. Bevorzugt als Rest X sind Chlor und Brom. Als Reste Y sind bevorzugt $-CH_3$, $-C_2H_5$, -Cl und -Br. Für Z wird der Rest $-COOR^2$ mit der Maßgabe bevorzugt, daß $R^2$ Methyl, Ethyl, iso-Propyl, n-, iso-Butyl, iso-Amyl, 2-Methoxy-ethyl und 1-Methoxy-2-propyl bedeuten.

Beispiele für geeignete Verbindungen Ic sind:

N-[3-(Acrylsäure-methylester)-4-chlorphenyl]-3,4,5,6-tetrahydrophthalimid
N-[3-(Acrylsäure-ethylester)-4-chlorphenyl]-3,4,5,6-tetrahydrophthalimid
N-[3-($\alpha$-Methyl-acrylsäure-methylester)-4-chlorphenyl]-3,4,5,6-tetrahydrophthalimid
N-[3-($\alpha$-Cyano-acrylsäure-methylester)-4-chlorphenyl]-3,4,5,6-tetrahydrophthalimid

N-[3-(α-Cyano-acrylsäure-ethylester)-4-chlorphenyl]-3,4,5,6,-tetrahydrophthalimid
N-[3-(α-Cyano-acrylsäure-iso-propylester)-4-chlorphenyl]-3,4,5,6,-tetrahydrophthalimid
N-[3-(α-Cyano-acrylsäure-n-butylester)-4-chlorphenyl]-3,4,5,6-tetrahydrophthalimid
N-[3-(α-Cyano-acrylsäure-iso-butylester)-4-chlorphenyl]-3,4,5,6-tetrahydrophthalimid
N-[3-(α-Cyano-acrylsäure-iso-amylester)-4-chlorphenyl]-3,4,5,6-tetrahydrophthalimid
N-[3-(α-Cyano-acrylsäure-benzylester)-4-chlorphenyl]-3,4,5,6,-tetrahydrophthalimid
N-[3-(α-Cyano-acrylsäure-cyclohexylester)-4-chlorphenyl]-3,4,5,6-tetrahydrophthalimid
N-[3-(α-Cyano-acrylsäure-2,2,2-trifluorethylester)-4-chlorphenyl]-3,4,5,6-tetrahydrophthalimid
N-[3-(α-Cyano-acrylsäure-2-methoxy-ethylester)-4-chlorphenyl]-3,4,5,6-tetrahydrophthalimid
N-[3-(α-Cyano-acrylsäure-allylester)-4-chlorphenyl]-3,4,5,6-tetrahydrophthalimid
N-[3-(α-Cyano-acrylsäure-3-methyl-allylester)-4-chlorphenyl]-3,4,5,6-tetrahydrophthalimid
N-[3-(α-Cyano-acrylsäure-2-chlorethylester)-4-chlorphenyl]-3,4,5,6-tetrahydrophthalimid
N-[3-(α-Cyano-acrylsäure-propargylester)-4-chlorphenyl]-3,4,5,6-tetrahydrophthalimid
N-[3-(α-Cyano-acrylsäure-1-methoxy-2-propylester)-4-chlorphenyl]-3,4,5,6-tetrahydrophthalimid
N-[3-(α-Brom-acrylsäure-methylester)-4-chlorphenyl]-3,4,5,6-tetrahydrophthalimid
N-[3-(α-Methyl-acrylsäure-ethylester)-4-chlorphenyl]-3,4,5,6-tetrahydrophthalimid
N-[3-(α-Methyl-acrylsäure-isobutylester)-4-chlorphenyl]-3,4,5,6-tetrahydrophthalimid
N-[3-(Acrylsäure-iso-propylester)-4-chlorphenyl]-3,4,5,6-tetrahydrophthalimid
N-[3-(Acrylsäure-n-butylester)-4-chlorphenyl]-3,4,5,6-tetrahydrophthalimid
N-[3-(Acrylsäure-iso-butylester)-4-chlorphenyl]-3,4,5,6-tetrahydrophthalimid
N-[3-(Acrylsäure-iso-amylester)-4-chlorphenyl]-3,4,5,6-tetrahydrophthalimid
N-[3-(Acrylsäure-benzylester)-4-chlorphenyl]-3,4,5,6-tetrahydrophthalimid
N-[3-(Acrylsäure-cyclohexylester)-4-chlorphenyl]-3,4,5,6-tetrahydrophthalimid
N-[3-(Acrylsäure-2,2,2-trifluorethylester)-4-chlorphenyl]-3,4,5,6-tetrahydrophthalimid
N-[3-(Acrylsäure-2-methoxy-ethylester)-4-chlorphenyl]-3,4,5,6-tetrahydrophthalimid
N-[3-(Acrylsäure-allylester)-4-chlorphenyl]-3,4,5,6-tetrahydrophthalimid
N-[3-(Acrylsäure-3-methyl-allylester)-4-chlorphenyl]-3,4,5,6-tetrahydrophthalimid
N-[3-(Acrylsäure-2-chlorethylester)-4-chlorphenyl]-3,4,5,6-tetrahydrophthalimid
N-[3-(Acrylsäure-propargylester)-4-chlorphenyl]-3,4,5,6-tetrahydrophthalimid
N-[3-(Acrylsäure-1-methoxy-2-propylester)-4-chlorphenyl]-3,4,5,6-tetrahydrophthalimid
N-[3-(α-Methyl-acrylsäure-2-methoxy-ethylester)-4-chlorphenyl]-3,4,5,6-tetrahydrophthalimid
N-[3-(α-Methyl-acrylsäure-dimethylamid)-4-chlorphenyl]-3,4,5,6-tetrahydrophthalimid
N-[3-(α-Methyl-acrylsäure-N-morpholid)-4-chlorphenyl]-3,4,5,6-tetrahydrophthalimid
N-[3-(α-Cyano-propionsäure-ethylester)-4-chlorphenyl]-3,4,5,6-tetrahydrophthalimid
N-[3-(α-Cyano-propionsäure-methylester)-4-chlorphenyl]-3,4,5,6-tetrahydrophthalimid
N-[3-(α-Cyano-propionsäure-amid)-4-chlorphenyl]-3,4,5,6-tetrahydrophthalimid
N-[3-(Propionsäure-methylester)-4-chlorphenyl]-3,4,5,6-tetrahydrophthalimid
N-[3-(Propionsäure-ethylester)-4-chlorphenyl]-3,4,5,6-tetrahydrophthalimid
N-[3-(α-Methyl-propionsäure-methylester)-4-chlorphenyl]-3,4,5,6-tetrahydrophthalimid
N-[3-(α-Methyl-propionsäure-ethylester)-4-chlorphenyl]-3,4,5,6-tetrahydrophthalimid
N-[3-(Methylmalonsäure-diethylester)-4-chlorphenyl]-3,4,5,6-tetrahydrophthalimid
N-[3-(Methylmalonsäure-dimethylester)-4-chlorphenyl]-3,4,5,6-tetrahydrophthalimid
N-[3-(1,3-Butadien-1,1-dicarbonsäure-dimethylester)-4-chlorphenyl]-3,4,5,6-tetrahydrophthalimid
N-[3-(1,3-Butadien-1-cyano-1-carbonsäure-ethylester)-4-chlorphenyl]-3,4,5,6-tetrahydrophthalimid
N-[3-(1,3-Butadien-1-cyano-1-carbonsäure-methylester)-4-chlorphenyl]-3,4,5,6-tetrahydrophthalimid
N-[3-(1,3-Butadien-1-cyano-1-carbonsäure-amid)-4-chlorphenyl]-3,4,5,6-tetrahydrophthalimid
N-[3-(1,3-Butadien-1-cyano-3-methyl-1-carbonsäure-ethylester)-4-chlorphenyl]-3,4,5,6-tetrahydrophthalimid
N-[3-(1,3-Butadien-1-cyano-3-methyl-1-carbonsäure-methylester)-4-chlorphenyl]-3,4,5,6-tetrahydrophthalimid
N-[3-(α-Brom-acrylsäure-methylester)-4-bromphenyl]-3,4,5,6-tetrahydrophthalamid
N-[3-(α-Brom-acrylsäure-ethylester)-4-bromphenyl]-3,4,5,6-tetrahydrophthalamid
N-[3-(α-Chlor-acrylsäure-ethylester)-4-bromphenyl]-3,4,5,6-tetrahydrophthalamid
N-[3-(α-Methyl-acrylsäure-ethylester)-4-bromphenyl]-3,4,5,6-tetrahydrophthalamid
N-[3-(α-Cyano-acrylsäure-methylester)-4-bromphenyl]-3,4,5,6-tetrahydrophthalamid
N-[3-(α-Carbonsäure-amid-acrylsäure-methylester)-4-bromphenyl]-3,4,5,6-tetrahydrophthalamid
N-[3-(Acrylsäure-methylester)-4-bromphenyl]-3,4,5,6-tetrahydrophthalamid
N-[3-(Acrylsäure-ethylester)-4-bromphenyl]-3,4,5,6-tetrahydrophthalamid
N-[3-(1,3-Butadien-1-cyano-1-carbonsäure-methylester)-4-bromphenyl]-3,4,5,6-tetrahydrophthalamid
N-[3-(1,3-Butadien-1-cyano-3-methyl-1-carbonsäure-ethylester)-4-bromphenyl]-3,4,5,6-tetrahydrophthalimid

N-[3-(α-Cyano-pentansäure-methylester)-4-chlorphenyl]-3,4,5,6-tetrahydrophthalimid
N-[3-(α-Cyano-pentansäure-ethylester)-4-chlorphenyl]-3,4,5,6-tetrahydrophthalimid
N-[3-(n-Propan-1,1-malonsäure-diethylester)-4-chlorphenyl]-3,4,5,6-tetrahydrophthalimid
N-[3-(1-Cyano-3-methyl-pentansäure-methylester)-4-chlorphenyl]-3,4,5,6-tetrahydrophthalimid
N-[3-(1-Cyano-3-methyl-pentansäure-ethylester)-4-chlorphenyl]-3,4,5,6-tetrahydrophthalimid
N-[3-(3-Methyl-iso-butan-1,1-malonsäure-diethylester)-4-chlorphenyl]-3,4,5,6-tetrahydrophthalimid

Die N-substituierten 3,4,5,6-Tetrahydrophthalimide der Formel Ic bzw. die sie enthaltenden herbiziden Mittel können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungformen richten sich nach den Verwendungszwecken; sie sollten in jedem Fall möglichst die feinste Verteilung der erfindungsgemäßen Wirkstoffe gewährleisten.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Chlorbenzol, Isophoron oder stark polare Lösungsmittel, wie N,N-Dimethylformamid, Dimethylsulfoxid, N-Methylpyrrolidon oder Wasser in Betracht.

Wäßrige Anwendungsformen können aus Emulsionskonzentraten, Dispersionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sufonsäuren, z.B. Lignin-, Phenol-, Naphthalin-und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Die Formulierungen enthalten im allgemeinen zwischen 0,1 und 95 Gew.%, vorzugsweise zwischen 0,5 und 90 Gew.%, Wirkstoff.

Die Applikation kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Jahreszeit, Zielpflanzen und Wachstumsstadium 0,01 bis 5,0, vorzugsweise 0,05 bis 0,5 kg/ha.

In Anbetracht des erfaßbaren Wirkungsspektrums zur Unkrautbekämpfung, der Verträglichkeit der Kulturpflanzen oder der erwünschten Beeinflussung des Wachstums derselben sowie angesichts der Vielfalt der Applikationsmethoden können die erfindungsgemäßen Verbindungen je nach Substitutionsmuster in einer großen Zahl von Kulturpflanzen eingesetzt werden.

In Betracht kommen beispeilsweise folgende Kulturen:

| Botanischer Name | Deutscher Name |
|---|---|
| Allium cepa | Küchenzwiebel |
| Ananas comosus | Ananas |
| Arachis hypogaea | Erdnuß |
| Asparagus officinalis | Spargel |
| Avena sativa | Hafer |
| Beta vulgaris spp. altissima | Zuckerrübe |
| Beta vulgaris spp. rapa | Futterrübe |
| Beta vulgaris spp. esculenta | Rote Rübe |
| Brassica napus var. napus | Raps |
| Brassica napus var. napobrassica | Kohlrübe |
| Brassica napus var. rapa | Weiße Rübe |
| Brassica rapa var. silvestris | Rüben |
| Camellia sinensis | Teestrauch |
| Carthamus tinctorius | Saflor - Färberdistel |
| Carya illinoinensis | Pekannußbaum |
| Citrus limon | Zitrone |
| Citrus maxima | Pampelmuse |
| Citrus reticulata | Mandarine |
| Citrus sinensis | Apfelsine, Orange |
| Coffea arabica (Coffea canephora, Coffea liberica) | Kaffee |
| Cucumis melo | Melone |
| Cucumis sativus | Gurke |
| Cynodon dactylon | Bermudagras |
| Daucus carota | Möhre |
| Elaeis guineensis | Ölpalme |
| Fragaria vesca | Erdbeere |
| Glycine max | Sojabohne |
| Gossypium hirsutum (Gossypium arboreum, Gossypium herbaceum, Gossypium vitifolium) | Baumwolle |
| Helianthus annuus | Sonnenblume |
| Helianthus tuberosus | Topinambur |
| Hevea brasiliensis | Parakautschukbaum |
| Hordeum vulgare | Gerste |
| Humulus lupulus | Hopfen |
| Ipomoea batatas | Süßkartoffeln |
| Juglans regia | Walnußbaum |
| Lactuca sativa | Kopfsalat |
| Lens culinaris | Linse |
| Linum usitatissimum | Faserlein |

| Botanischer Name | Deutscher Name |
|---|---|
| Lycopersicon lycopersicum | Tomate |
| Malus spp. | Apfel |
| Manihot esculenta | Maniok |
| Medicago sativa | Luzerne |
| Mentha piperita | Pfefferminze |
| Musa spp. | Obst- und Mehlbanane |
| Nicotiana tabacum (N. rustica) | Tabak |
| Olea europaea | Ölbaum |
| Oryza sativa | Reis |
| Panicum miliaceum | Rispenhirse |
| Phaseolus lunatus | Mondbohne |
| Phaseolus mungo | Erdbohne |
| Phaseolus vulgaris | Buschbohnen |
| Pennisetum glaucum | Perl- oder Rohrkolbenhirse |
| Petroselinum crispum spp. tuberosum | Wurzelpetersilie |
| Picea abies | Rotfichte |
| Abies alba | Weißtanne |
| Pinus spp. | Kiefer |
| Pisum sativum | Gartenerbse |
| Prunus avium | Süßkirsche |
| Prunus domestica | Pflaume |
| Prunus dulcis | Mandelbaum |
| Prunus persica | Pfirsich |
| Pyrus communis | Birne |
| Ribes sylvestre | Rote Johannisbeere |
| Ribes uva-crispa | Stachelbeere |
| Ricinus communis | Rizinus |
| Saccharum officinarum | Zuckerrohr |
| Secale cereale | Roggen |
| Sesamum indicum | Sesam |
| Solanum tuberosum | Kartoffel |
| Sorghum bicolor (s. vulgare) | Mohrenhirse |
| Sorghum dochna | Zuckerhirse |
| Spinacia oleracea | Spinat |
| Theobroma cacao | Kakaobaum |
| Trifolium pratense | Rotklee |
| Triticum aestivum | Weizen |
| Vaccinium corymbosum | Kulturheidelbeere |
| Vaccinium vitis-idaea | Preißelbeere |
| Vicia faba | Pferdebohnen |
| Vigna sinensis (V. unguiculata) | Kuhbohne |
| Vitis vinifera | Weinrebe |
| Zea mays | Mais |

Zur Verbreiterung des Wirkungsspektrums und zur Erzielung synergistischer Effekte können die Tetrahydrophthalimide der Formel Ic mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, andere Harnstoffe, Diphenylether, Triazinone, Uracile, Benzofuranderivate, Cyclohexan-1,3-dionderivate und andere in Betracht.

Außerdem kann es von Nutzen sein, die neuen Verbindungen der Formel Ic allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

Beispiele

Herstellung von Nitrobenzolderivaten Ia

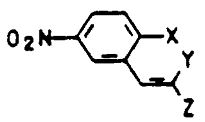

(Ia)

Beispiel 1

2-Chlor-5-nitro-zimtsäure (Y = H; Z = COOH)

185,5 g (1 mol) 2-Chlor-5-nitrobenzaldehyd und 125 g (1,2 mol) Malonsäure in 400 ml Pyridin wurden mit 8,5 g Piperidin versetzt und bis zur Beendigung der $CO_2$-Entwicklung (ca. 3 h) bei 110 °C gerührt. Das Reaktionsgemisch wurde danach auf eine Mischung aus 1 l Eis und 350 ml konz. HCl gegossen, wobei ein gelber Feststoff ausfiel. Nach Absaugen des Produktes, Waschen mit Wasser und Trocknen erhielt man 220 g (97 % d.Th.) der oben genannten Verbindung mit Fp. 193-196 °C.

Beispiel 2

2-Chlor-5-nitro-zimtsäure-methylester (Y = H; Z = $COOCH_3$)

Eine Lösung von 34,1 g (150 mmol) des Produktes aus Beispiel 1 in 300 ml Methanol wurde mit HCl-Gas gesättigt, wonach weiteres HCl-Gas während der 5 stündigen Reaktionszeit eingeleitet wurde. Der beim Erkalten erhaltene Feststoff wurde mit $NaHCO_3$-Wasser durchgerührt und in Methylenchlorid aufgenommen. Nach Waschen der $CH_2Cl_2$-Lösung mit Wasser und Trocknen über $MgSO_4$ wurde das o.g. Produkt durch Eindampfen und anschließendes Anreiben mit Diethylether gewonnen. Ausbeute: 24 g (63 %), Fp. 159-162 °C.

Beispiel 3

2-Chlor-5-nitro-zimtsäure-ethylester (Y = H; Z = $COOC_2H_5$)

Analog Beispiel 1 wurden 33 g (250 mmol) Malonsäuremonoethylester und 27 g (150 mmol) 2-Chlor-5-nitrobenzaldehyd in 100 ml Pyridin umgesetzt und wie üblich aufgearbeitet. Die Ausbeute betrug 31 %; Fp. 138-140 °C.

Beispiel 4

3-Nitro-zimtsäure-ethylester (X = H; Y = H; Z = $COOC_2H_5$)

Die Umsetzung und Aufarbeitung erfolgte wie in Beispiel 3 und erbrachte das Produkt in 72 %iger Ausbeute; Fp. 67-70 °C.

Beispiel 5

3-Nitro-zimtsäure-methylester (X = H; Y = H; Z = $COOCH_3$)

Analog Beispiel 4 wurde gearbeitet, und man erhielt das Produkt in 74 %iger Ausbeute; Fp. 101-103 °C.

Biespiel 6

2-Chlor-5-nitro-α-methyl-zimtaldehyd (Y = $CH_3$; Z = CHO)

Zu einer Suspension von 185,5 g (1 mol) 2-Chlor-5-nitrobenzaldehyd in 500 ml Methanol wurden 4 g NaOH, gelöst in 20 ml Wasser, zugegeben, wonach unter Kühlung auf 10 bis 15 °C 70 g (1,2 mol) Propionaldehyd zugetropft wurden. Nach 2 tägigem Rühren bei Raumtemperatur wurde mit Eisessig neutralisiert und der ausgefallene Feststoff durch Absaugen isoliert. Nach Waschen mit Wasser und Trocknen bei 50 °C im Vakuum erhielt man das Produkt in 78 %iger Ausbeute; Fp. 99 bis 102 °C.

Beispiel 7

2-Chlor-5-nitro-α-methyl-zimtsäure (Y = $CH_3$; Z = COOH)

Man mischte 371 g (2 mol) 2-Chlor-5-nitro-benzaldehyd mit 192 g (2 mol) Propionsäure-natriumsalz und 780 g (6 mol) Propionsäureanhydrid. Dann wurde unter Rühren 6 Stunden auf 140 bis 160 °C erhitzt, wobei Lösung erfolgte. Danach ließ man auf 90 °C abkühlen, versetzte mit 200 ml Eiswasser und isolierte den ausgefallenen Feststoff durch Absaugen. Nach Waschen mit Wasser wurde aus Aceton umkristallisiert. Ausbeute: 318g (66 % d.Th.) mit Fp. 228 bis 230 °C.

Beispiel 8

2-Chlor-5-nitro-α-methyl-zimtsäure-methylester (Y = $CH_3$; Z = $COOCH_3$)

Durch Umsetzung der in Beispiel 7 erhaltenen Säure in bekannter Weise mit Thionylchlorid in Toluol entstand das Säurechlorid, das in überschüssigem Methanol zum Rückfluß erhitzt wurde und den Ester in 72 %iger Ausbeute lieferte; Fp. 98 bis 99 °C.

Beispiel 9

2-Chlor-5-nitro-α-cyano-zimtsäure-ethylester (Y = CN; Z-$COOC_2H_5$)

Zu einer Lösung von 55,7 g (300 mmol) 2-Chlor-5-nitrobenzaldehyd in 200 ml Tetrahydrofuran wurden 29,7 g (300 mmol) Cyanessigsäure-ethylester gegeben, wonach bei 0 °C 1 ml Piperidin als Katalysator zugefügt wurde. Nach 16 Stunden Rühren bei Raumtemperatur wurde mit Essigsäure neutralisiert und wie üblich aufgearbeitet. Fp. 83 bis 85 °C, Ausbeute: 71 %.

Beispiel 10

3-Nitro-benzyliden-malonsäure-dimethylester (X = H; Y und Z = $COOCH_3$)

Analog Beispiel 9 wurden 75,5 g (500 mmol) 3-Nitrobenzaldehyd und 73 g (550 mmol) Malonsäuredimethylester 4 Stunden bei 60 °C gerührt. Nach Zugabe von 5 ml Eisessig erfolgte die Aufarbeitung wie üblich. In 96 %iger Ausbeute entstand das Produkt; Fp. 78 bis 80 °C.

Beispiel 11

1-(2-Chlor-5-nitrophenyl)-4-cyano-4-methoxycarbonyl-trans-buta-1,3-dien (Y = H; Z = -CH = C(CN)$COOCH_3$)

15 g (70 mmol) 2-Chlor-5-nitro-zimtaldehyd (Fp. 115-117 °C) und 10 g (0,1 mol) Cyanessigsäuremethylester wurden in 200 ml Toluol am Wasserabscheider in Gegenwart von 1 g Ammoniumacetat und 1 ml Essigsäure erhitzt, bis kein Wasser mehr überdestillierte. Das Produkt kristallisierte beim Abkühlen aus.

Nach der üblichen Aufarbeitung verblieben 58 % des Produktes mit Fp. 193 bis 194 °C.

Herstellung von Anilinderivaten Ib

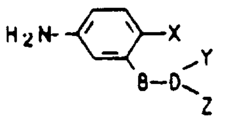

(Ib)

Beispiele 12 bis 18

Herstellung von Anilinderivaten Ib mit ungesättigten Seitenketten

Einer Suspension von 3,3 Äquivalenten Eisenpulver in einem Gemisch von 100 ml desjenigen Alkohols, welcher der Estergruppe in Z entspricht und 25 ml Eisessig wurde bei 50 °C eine unter Erwärmen hergestellte Lösung von 1 Äquivalent einer Nitroverbindung Ia in 50 ml Alkohol und 50 ml Eisessig zugefügt und 3 Stunden zum Sieden erhitzt. Die Aufarbeitung erfolgte durch Abfiltrieren, Eingießen des Filtrats in 500 ml Wasser und Extraktion mit Essigsäureethylester oder Methylenchlorid. Nach Trochnung der organischen Phase und Abdestillation des Lösungsmittels im Vakuum wurde das Produkt ohne weitere Reinigung gemäß Beispiel 22 mit Tetrahydrophthalsäureanhydrid kondensiert. Weitere Eigenschaften sind in Tabelle 1 zusammengefaßt.

Tabelle 1

Verbindungen 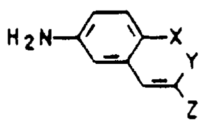

| Bsp. Nr. | Z | Y | X | Fp. °C | Ausbeute % |
|---|---|---|---|---|---|
| 12 | $COOCH_3$ | H | Cl | 70-72 | 91 |
| 13 | $COOC_2H_5$ | H | Cl | 68-70 | 84 |
| 14 | $COOCH_3$ | $CH_3$ | Cl | 82-84 | 86 |
| 15 | $COOCH_3$ | CN | Cl | 88-90 | 89 |
| 16 | $COOC_2H_5$ | CN | Cl | 83-85 | 88 |
| 17 | $-CH{=}C(CN)(COOCH_3)$ | H | Cl | 127-129 | 90 |
| 18 | $COOCH_3$ | H | H | 58-59 | 88 |

Beispiele 19 bis 21

Herstellung von Anilinderivaten Ib mit gesättigter Seitenkette

0,2 mol einer Nitroverbindung Ia wurden in Gegenwart von 5 g 10 % Pd/C bei 20 bis 40 °C unter Normaldruck in 500 ml Tetrahydrofuran bzw. Essigsäureethylester hydriert und wie üblich aufgearbeitet. Die Ergebnisse sind Tabelle 2 zu entnehmen.

Tabelle 2

Verbindungen 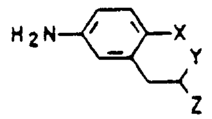

| Bsp. Nr. | Z | Y | X | Fp. °C | Ausbeute % |
|---|---|---|---|---|---|
| 19 | $COOC_2H_5$ | H | H | Öl | 83 |
| 20 | $COOC_2H_5$ | CN | Cl | Öl | 85 |
| 21 | $COOCH_3$ | $COOCH_3$ | H | Öl | ~ 100 |

Herstellung von N-substituierten 3,4,5,6-Tetrahydrophthalimiden Ic

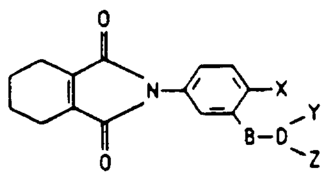

(Ic)

Allgemeine Arbeitsvorschrift

Man erhitzt eine Mischung von 0,1 mol 3,4,5,6-Tetrahydrophthalsäureanhydrid und 0,1 mol eines Anilins der allgemeinen Formel Ib in 150 ml Essigsäure solange zum Sieden, bis die Komponenten nach DC umgesetzt sind, was nach 2 bis 3 Std. der Fall ist. Das Produkt fällt meist beim Abkühlen aus und wird dann durch Absaugen isoliert. Wenn das nicht der Fall ist, wird das Reaktionsgemisch im Vakuum eingedampft, in Essigsäureethylester gelöst und mit Wasser extrahiert. Nach Trocknen und Abziehen des Essigesters verbleibt das Produkt als Öl, das durch Anreiben mit Petrolether auskristallisiert. Die physikalischen Eigenschaften der Wirkstoffe sind in den Tabellen 3,4 und 5 zusammengefaßt.

Bei den ungesättigten Verbindungen (Tabelle 3) treten cis-trans-Isomere in wechselndem Verhältnis auf. Beide Isomere sowohl wie ihr Gemisch werden beansprucht.

Beispiel 22

N-[3-(α-Cyano-propionsäure-ethylester)-4-chlorphenyl]-3,4,5,6-tetrahydrophthalimid

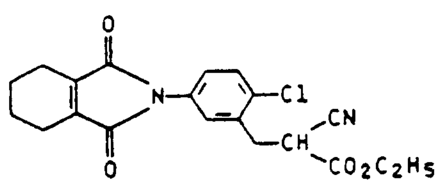

14 g (55 mmol) des entsprechenden gesättigten Anilinderivats gemäß Beispiel 18 und 9 g (60 mmol) 3,4,5,6-Tetrahydrophthalsäureanhydrid wurden mit 200 ml Xylol solange am Wasserabscheider zum Rückfluß erhitzt, bis kein Wasser mehr überging. Nach der üblichen Aufarbeitung und Chromatographie mit Toluol/Tetrahydrofuran = 7 + 3 an Kieselgel erhielt man das Produkt in 61 %iger Ausbeute als Öl.

Beispiel 23

N-[3-(Propionsäure-ethylester)-4-chlorphenyl]-3,4,5,6-tetrahydrophthalimid

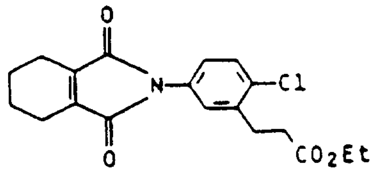

16 g (49 mmol) N-[3-(Propionsäure-ethylester)-phenyl]-3,4,5,6-tetrahydrophthalimid wurden in 150 ml Eisessig gelöst. 7,3 g (54 mmol) Sulfurylchlorid, in 20 ml Eisessig gelöst, wurden bei 80 °C zugetropft, wonach 2 Stunden bei 110 °C gerührt wurde. Nach der üblichen Aufarbeitung wurde das Rohprodukt durch Chromatographie an Kieselgel (Cyclohexan/Essigester = 1 + 1) gereinigt. Man erhielt das Produkt als Öl in 54 %iger Ausbeute.

Herstellung von N-substituierten 3,4,5,6-Tetrahydrophthalimiden Ic mit halogenhaltiger Seitenkette

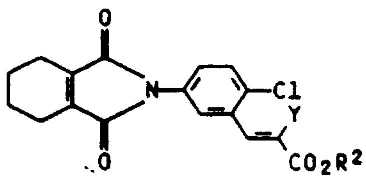

Y = Halogen

N-[3-(α,β-Dibrompropionsäuremethylester)-4-chlorphenyl]-3,4,5,6-tetrahydrophthalimid als Zwischenprodukt

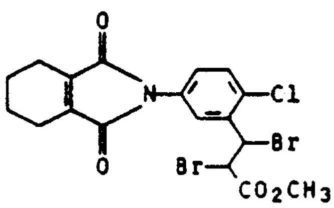

Zu einer Lösung von 10 g (30 mmol) des Wirkstoffs 1 aus Tabelle 3 in 50 ml Methylenchlorid wurde bei einer Badtemperatur von 50 °C eine Lösung von 4,6 g (30 mmol) Brom in 10 ml Methylenchlorid zugetropft. Nach 2 stündigem Rühren war die Reaktion beendet. Das Produkt wurde meist ohne weitere Isolierung zur Dehydrobromierung gemäß Beispiel 25 eingesetzt. Eine durch Abdampfen von Methylenchlorid isolierte Probe zeigte Fp. 90 bis 94 °C.

Beispiel 24

N-[3-(α-Bromacrylsäure-methylester)-4-chlorphenyl]-3,4,5,6-tetrahydrophthalimid

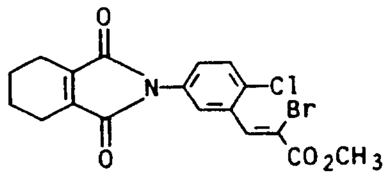

Das wie oben beschrieben erhaltene Dibromid wurde anschließend direkt in 150 ml Methylenchlorid bei Raumtemperatur mit 10,1 g (100 mmol) Triethylamin durch einstündiges Rühren bei 25 °C dehydrobromiert. Nach 15 min Aufkochen wurde das Reaktionsgemisch auf Wasser gegossen, die Methylenchloridphase wurde abgetrennt, mit Wasser gewaschen, getrocknet und eingedampft. Nach Anreiben mit Diethylether wurden 10 g (85 %) eines Feststoffs mit Fp. 92 bis 94 °C erhalten.

Beispiel 25

N-[3-(α-Bromacrylsäuremethylester)-4-bromphenyl]-3,4,5,6-tetrahydrophthalimid

a) 2-Brom-5-nitrobenzaldehyd

Zu einer Lösung von 63,4 g (0,34 mol) 2-Brombenzaldehyd in 110 ml konz. $H_2SO_4$ wurde bei -5 °C eine Nitriersäure getropft, die bei -10 °C durch Zugabe von 55 ml konz. $H_2SO_4$ zu 21 ml 98 proz. $HNO_3$ hergestellt worden war. Die Zutropfdauer betrug 60 min. Nach 45 min zusätzlichem Rühren wurde die Reaktionsmischung auf 550 g Eis gegossen, der ausfallende Feststoff wurde abgesaugt, mit Wasser, dann mit $Na_2CO_3$-Lösung (10 proz.) und erneut mit Wasser gewaschen. Nach Trocknen bei 50 °C i.Vak. erhielt man 72 g (92 % d.Th.) als gelben Feststoff; Fp. 86-88 °C.

b) 2-Brom-5-nitro-zimtsäure

Diese Reaktion wurde analog Beispiel 1 mit 2-Brom-5-nitrobenzaldehyd und Malonsäure in Pyridin mit Piperidin durchgeführt. Man erhielt das Produkt in 57 %iger Ausbeute; Fp. 185 °C.

c) 2-Brom-5-nitrozimtsäuremethylester

Dieses Produkt wurde analog Beispiel 8 durch Überführung der unter b) erhaltenen Zimtsäure in das Säurechlorid (Fp. 106-108 °C, 98 % Ausbeute) und anschließende Umsetzung mit Methanol/Pyridin hergestellt. Ausbeute 70 % d.Th., Fp. 150-152 °C.

d) 2,α-Dibrom-5-nitro-zimtsäuremethylester

Zu einer Lösung von 28,6 g (0,1 mol) des unter c) erhaltenen Esters in 200 ml Methylenchlorid tropfte man bei 40 °C innerhalb 2 1/2 Std. 19,2 g (0,12 mol) Brom; anschließend wurde noch 3 Std. zum Sieden erhitzt. Nach dem Abkühlen auf 20 °C wurden 20,2 g (0,2 mol) Triethylamin zugetropft. Nach 3 Std. wurde die Reaktionsmischung mit 150 ml Wasser versetzt und in einen Schütteltrichter überführt. Nach 2maliger Extraktion der Methylenchloridphase mit je 100 ml Wasser wurde die org. Phase getrocknet ($MgSO_4$) und i.Vak. eingeengt. Nach Anreiben mit Diisopropylether erhielt man 30 g eines Rohproduktes, das über Kieselgel mit dem Laufmittel Toluol chromatographiert wurde. Nach Einengen der Toluollösung erhielt man 20 g (56 %) des Produkts mit Fp. 95-96 °C.

e) 2,α-Dibrom-5-amino-zimtsäuremethylester

Zu einer Suspension von 15.7 g (0,28 mol) Eisen in 200 ml Methanol und 100 ml Eisessig bei 60 °C wurden portionsweise 20 g (0,056 mol) der unter d) hergestellten Nitroverbindung gegeben. Nach 5 Stunden Refluxieren wurde abgekühlt, in 1,5 l Wasser gegossen und 2 × mit 200 ml Essigsäureethylester extrahiert. Die org. Phase wurde mit Wasser gewaschen. getrocknet und i.Vak. eingeengt. Man erhielt das Produkt (18g ≙ 96 % d.Th.) als Öl.

f) N-[3-(α-Bromacrylsäuremethylester)-4-bromphenyl]-3,4.5,6-tetrahydrophthalimid

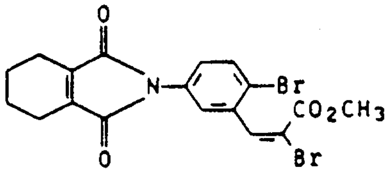

(cis)

Wirkstoff Nr. 67

Zu einer Lösung von 18 g (0,053 mol) des unter e) erhaltenen Produkts in 50 ml Eisessig wurden 8,1 g (0,053 mol) Tetrahydrophthalsäureanhydrid gegeben. Nach 3 Std. Rühren bei 110 °C wurde i.Vak. eingeengt, der Rückstand in Essigsäureethylester gelöst und mit 10 % $NaHCO_3$-Lösung sowie mit Wasser extrahiert. Nach Trocknen der Essigesterphase mit $MgSO_4$ wurde i.Vak. eingedampft. Nach Anreiben mit Diisopropylether erhielt man 23 g (92 % d.Th.) des Produktes mit Fp. 83-85 °C.
Die NMR-Analyse zeigte, daß die Verbindung zu 85 % in der cis-Form und zu 15 % in der trans-Form vorlag.

Beispiel 26

N-[3-(α-Chloracrylsäuremethylester)-4-chlorphenyl]-3,4,5,6-tetrahydrophthalimid

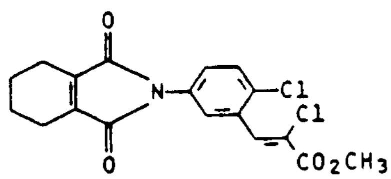

Wirkstoff Nr. 49

a) 3-[2'-Chlorphenyl]-2,3-dichlorpropionsäuremethylester
Zu einer Lösung von 39,3 g (0,2 mol) 2'-Chlorphenyl-zimtsäuremethylester in 200 ml 1,1,1-Trichlorethan wurden bei 60 °C innerhalb 3 Std. langsam 21,3 g (0,3 mol) Chlorgas eingeleitet und anschließend noch 1 Std. bei 70 °C gerührt. Dann wurde die Reaktionsmischung 2 mal mit 150 ml Wasser gewaschen und die org. Phase anschließend uber $MgSO_4$ getrocknet. Nach Abziehen des Lösungsmittels i.Vak. erhielt man 52 g (97 %) des Produktes als Öl.
b) 2,α-Dichlorzimtsäuremethylester
Zu einer Lösung von 52 g (0,194 mol) des under a) erhaltenen Esters in 400 ml Methylenchlorid wurden bei 25 °C 40,4 g (0,4 mol) Triethylamin getropft und anschließend 1 Std. bei 40 °C gerührt. Danach wurden 200 ml Wasser zugefügt und die org. Phase abgetrennt. Nach Waschen der $CH_2Cl_2$-Lösung mit Wasser und Trocknen mit $MgSO_4$ erhielt man 42 g (93 % d.Th.) des Produktes als Öl.
c) 2,α-Dichlor-5-nitrozimtsäuremethylester
Man bereitete eine Nitriersäure durch Zutropfen von 47 ml konz. $H_2SO_4$ zu 16 ml 98 proz. $HNO_3$ bei -10 °C und tropfte diese zu einer Lösung von 42 g (0,182 mol) des unter b) beschriebenen α-Chlorzimtsäureesters in 150 ml konz. $H_2SO_4$ bei -5 °C. Nach 30 min Nachrühren bei 0 °C wurde die Reaktionsmischung in 600 ml Eiswasser gerührt und das ausfallende Produkt durch Absaugen isoliert. Nach mehrmaligem Waschen mit Wasser wurde das noch feuchte Produkt aus Methanol umkristallisiert. Man erhielt 28g (54 % d.Th.) Kristalle mit Fp. 108-110 °C.
d) 2,α-Dichlor-5-amino-zimtsäuremethylester
100 g (0,36 mol) des unter c) beschriebenen Nitroproduktes wurden mit 61,6 g (1,1 mol) Eisenpulver in 500 ml Methanol und 500 ml Eisessig wie bei Beispiel 25e reduziert. Nach der dort beschriebenen Aufarbeitung wurden 70 g (80 % d.Th.) des gewünschten Produktes in Form gelber Kristalle erhalten. Fp. 104-105 °C.

e) Tetrahydrophthalimidderivat

12,3 g (0,05 mol) des unter d) hergestellten Anilinderivates wurden in 50 ml Eisessig mit 7,6 g (0,05 mol) Tetrahydrophthalsäureanhydrid versetzt und 3 Std. bei 110 °C gerührt. Nach Abziehen der Essigsäure i.Vak. wurde der Rückstand in Essigsäureethylester aufgenommen und mit 10 % $NaHCO_3$-Lösung, danach mit Wasser gewaschen. Nach Trocknen der org. Phase ($MgSO_4$) und Abziehen des Lösungsmittels erhielt man 13 g (68 % d.Th.) weißer Kristalle mit Fp. 138-140 °C. Nach NMR liegt die Verbindung als reines trans-Isomeres vor.

Nach den vorstehend angegebenen Methoden wurden die in den Tabellen 3 bis 5 zusammengestellten Wirkstoffe erhalten.

Tabelle 3

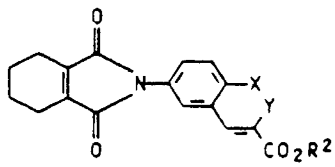

| Lfd. Nr. | X | Y | $R^2$ | Fp. (°C) |
|---|---|---|---|---|
| 1 | Cl | H | $CH_3$ | 138-140 |
| 2 | Cl | H | $C_2H_5$ | 148-149 |
| 3 | Cl | H | $C_3H_7$-i | 106-107 |
| 4 | Cl | H | $C_4H_9$-n | 74- 76 |
| 5 | Cl | H | $C_4H_9$-i | 52- 53 |
| 6 | Cl | H | $C_5H_{11}$-i | 104-106 |
| 7 | Cl | H | Cyclohexyl | 131-133 |
| 8 | Cl | H | 2-Methoxy-ethyl | 76- 78 |
| 9 | Cl | H | 1-Methoxy-propyl-2 | 30- 31 |
| 10 | Cl | H | Allyl | 110-111 |
| 11 | Cl | H | 3-Methylallyl | 102-103 |
| 12 | Cl | $CH_3$ | H | 212-213 |
| 13 | Cl | $CH_3$ | $CH_3$ | 132-134 |
| 14 | Cl | $CH_3$ | $C_2H_5$ | 62- 63 |
| 15 | Cl | $CH_3$ | $C_3H_7$-n | 79- 80 |
| 16 | Cl | $CH_3$ | $C_3H_7$-i | 74- 75 |
| 17 | Cl | $CH_3$ | $C_4H_9$-n | 84- 86 |
| 18 | Cl | $CH_3$ | $C_4H_9$-i | 86- 88 |
| 19 | Cl | $CH_3$ | $C_5H_{11}$-n | 47- 49 |
| 20 | Cl | $CH_3$ | $C_5H_{11}$-i | 64- 65 |
| 21 | Cl | $CH_3$ | Benzyl | 76- 78 |
| 22 | Cl | $CH_3$ | 2-Phenylethyl | Öl |
| 23 | Cl | $CH_3$ | 1-Phenyl-propyl-1 | 108-109 |
| 24 | Cl | $CH_3$ | Propargyl | 151-152 |
| 25 | Cl | $CH_3$ | 3-Methylbuten-2-yl | 49- 51 |
| 26 | Cl | $CH_3$ | 3-Methylbuten-3-yl | Öl |
| 27 | Cl | $CH_3$ | 2-Methoxyethyl | 41- 44 |
| 28 | Cl | $CH_3$ | 2-Ethoxyethyl | 39- 40 |
| 29 | Cl | $CH_3$ | 2-(n-Butoxy)-ethyl | Öl |
| 30 | Cl | $CH_3$ | 1-Methoxy-propyl-2 | 45- 47 |

| Lfd. Nr. | X | Y | $R^2$ | Fp. (°C) |
|---|---|---|---|---|
| 31 | Cl | $CH_3$ | 2-Methoxy-propyl-1 | 89- 90 |
| 32 | Cl | $CH_3$ | 1-Ethoxy-propyl-2 | Öl |
| 33 | Cl | $CH_3$ | 1-Methoxy-butyl-2 | 55- 56 |
| 34 | Cl | $CH_3$ | 2-Ethyl-thio-ethyl | 39- 40 |
| 35 | Cl | $CH_3$ | 2-(Isopropylthio)-ethyl | 67- 68 |
| 36 | Cl | $C_2H_5$ | $CH_3$ | 103-104 |
| 37 | Cl | $C_2H_5$ | $C_2H_5$ | 49- 50 |
| 38 | Cl | $C_2H_5$ | 2-Methoxyethyl | 46- 48 |
| 39 | Cl | $C_2H_5$ | 1-Methoxypropyl-2 | 32- 33 |
| 40 | Cl | $C_2H_5$ | $n-C_3H_7$ | 47- 48 |
| 41 | Cl | $C_2H_5$ | $n-C_4H_9$ | Öl |
| 42 | Cl | $i-C_3H_7$ | $CH_3$ | 75- 76 |
| 43 | Cl | $n-C_3H_7$ | $CH_3$ | Öl |
| 44 | Cl | $n-C_3H_7$ | 2-Methoxyethyl | Öl |
| 45 | Cl | $n-C_4H_9$ | $CH_3$ | Öl |
| 46 | Cl | $n-C_4H_9$ | 2-Methoxyethyl | Öl |
| 47 | Cl | $n-C_5H_{11}$ | $CH_3$ | Öl |
| 48 | Cl | $n-C_5H_{11}$ | 2-Methoxyethyl | Öl |
| 49 | Cl | Cl | $CH_3$ | 138-140 |
| 50 | Cl | Cl | $C_2H_5$ | 87- 89 |
| 51 | Cl | Cl | $n-C_3H_7$ | 83- 85 |
| 52 | Cl | Cl | $i-C_3H_7$ | 140-141 |
| 53 | Cl | Cl | $n-C_4H_9$ | 90- 91 |
| 54 | Cl | Cl | $n-C_5H_{11}$ | 112-114 |
| 55 | Cl | Cl | $i-C_5H_{11}$ | 68- 70 |
| 56 | Cl | Cl | 2-Methoxyethyl | 128-130 |
| 57 | Cl | Cl | 2-Ethoxyethyl | 58- 60 |
| 58 | Cl | Cl | 2-(n-Butoxy)-ethyl | Öl |
| 59 | Cl | Br | $CH_3$ | 94- 95 |
| 60 | Cl | Br | $C_2H_5$ | 91- 92 |
| 61 | Cl | Br | $n-C_3H_7$ | 78- 80 |
| 62 | Cl | Br | $i-C_3H_7$ | 119-121 |
| 63 | Cl | Br | $n-C_4H_9$ | 73- 74 |
| 64 | Cl | Br | 2-Methoxyethyl | 136-137 |
| 65 | Br | H | $CH_3$ | 132-133 |
| 66 | Br | H | $n-C_4H_9$ | 121-122 |

| Lfd. Nr. | X | Y | $R^2$ | Fp. (°C) |
|---|---|---|---|---|
| 67 | Br | Br | $CH_3$ | 83- 85 |
| 68 | Cl | CN | H | 148-151 |
| 69 | Cl | CN | $CH_3$ | 154-155 |
| 70 | Cl | CN | $C_2H_5$ | 140-142 |
| 71 | Cl | CN | $i-C_3H_7$ | 132-133 |
| 72 | Cl | CN | $i-C_4H_9$ | 134-135 |
| 73 | Cl | CN | Benzyl | 182-183 |
| 74 | Cl | CN | 2-Methoxyethyl | 114-116 |
| 75 | Cl | CN | 2-Isopropoxyethyl | 63- 65 |
| 76 | Cl | CN | 1-Methoxypropyl-2 | 56- 57 |
| 77 | Cl | CN | 2-Methoxypropyl-1 | 120-123 |
| 78 | Cl | $CO_2CH_3$ | $CH_3$ | 138-140 |
| 79 | H | H | $CH_3$ | 141-142 |

Tabelle 4

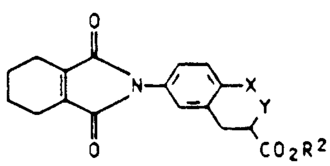

| Lfd. Nr. | X | Y | $R^2$ | Fp. (°C) |
|---|---|---|---|---|
| 80 | Cl | H | $C_2H_5$ | Öl |
| 81 | Cl | CN | $C_2H_5$ | Öl |
| 82 | Cl | $CO_2CH_3$ | $CH_3$ | Öl |

Tabelle 5

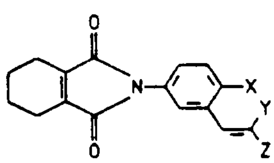

| Lfd. Nr. | X | Y | Z | Fp. (°C) |
|---|---|---|---|---|
| 83 | Cl | $CH_3$ | CN | 112-113 |
| 84 | Cl | H | 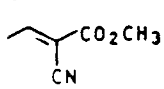 | 176-179 |
| 85 | Cl | H | 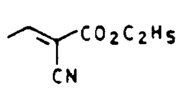 | 184-186 |

Analog können folgende Wirkstoffe der Formel I erhalten werden:

Tabelle 6

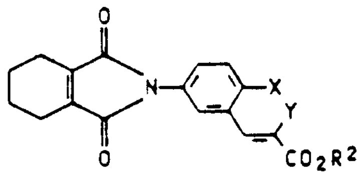

| Nr. | X | Y | $R^2$ |
|---|---|---|---|
| 101 | Cl | $CH_3$ | Allyl |
| 102 | Cl | $CH_3$ | 2-Methyl-thioethyl |
| 103 | Cl | $CH_3$ | Cyclohexyl |
| 104 | Cl | $CH_3$ | Cyclopentyl |
| 105 | Cl | $CH_3$ | 2-Chlorethyl |
| 106 | Cl | $CH_3$ | 2,2,2-Trifluorethyl |
| 107 | Cl | $C_2H_5$ | i-$C_3H_7$ |
| 108 | Cl | $C_2H_5$ | i-$C_4H_9$ |
| 109 | Cl | $C_2H_5$ | n-$C_5H_{11}$ |
| 110 | Cl | $C_2H_5$ | i-$C_5H_{11}$ |
| 111 | Cl | $C_2H_5$ | 2-Ethoxyethyl |
| 112 | Cl | $C_2H_5$ | 2-(n-Butoxy)-ethyl |
| 113 | Cl | $C_2H_5$ | Cyclohexyl |
| 114 | Cl | $C_2H_5$ | Cyclopentyl |
| 115 | Cl | $C_2H_5$ | 2-Chlorethyl |
| 116 | Cl | Cl | 2-Chlorethyl |
| 117 | Cl | Cl | i-$C_4H_9$ |
| 118 | Cl | Cl | sek.-$C_4H_9$ |
| 119 | Cl | Br | i-$C_4H_9$ |
| 120 | Cl | Br | n-$C_5H_{11}$ |
| 121 | Cl | Br | n-$C_6H_{13}$ |
| 122 | Cl | Br | 2-Chlorethyl |
| 123 | Cl | Br | i-$C_5H_{11}$ |
| 124 | Cl | Br | Cyclohexyl |
| 125 | Cl | Br | Cyclopentyl |
| 126 | Br | Br | $C_2H_5$ |
| 127 | Br | Br | n-$C_3H_7$ |
| 128 | Br | Br | 2-Methoxyethyl |

| Nr. | X | Y | $R^2$ |
|---|---|---|---|
| 129 | Br | Br | 2-Ethoxyethyl |
| 130 | Br | Cl | $CH_3$ |
| 131 | Br | Cl | $C_2H_5$ |
| 132 | Br | Cl | n-$C_3H_7$ |
| 133 | Br | Cl | 2-Methoxyethyl |
| 134 | Br | Cl | 2-Ethoxyethyl |
| 135 | Cl | $C_2H_5$ | Allyl |
| 136 | Cl | $C_2H_5$ | 3-Methylallyl |
| 137 | Cl | Cl | Allyl |
| 138 | Cl | Cl | 3-Methylallyl |
| 139 | Br | Cl | Allyl |
| 140 | Cl | Br | 3-Methylallyl |
| 141 | Br | Br | Allyl |
| 142 | Br | Br | 3-Methylallyl |
| 143 | Br | Br | 2-Chlorethyl |
| 144 | Br | Cl | Allyl |
| 145 | Br | Cl | 3-Methylallyl |
| 146 | Br | Cl | 2-Chlorethyl |

Anwendungsbeispiele

Die Wirkung der N-substituierten 3,4,5,6-Tetrahydrophthalimide der Formel Ic auf das Wachstum der Testpflanzen ließ sich durch folgende Gewächshausversuche zeigen.

Als Kulturgefäße dienten Plastikblumentöpfe mit 300 $cm^3$ Inhalt und lehmigem Sand mit etwa 3 % Humus als Substrat. Die Samen der Testpflanzen wurden nach Arten getrennt flach eingesät.

Zum Zwecke der Nachauflaufbehandlung wurden entweder direkt gesäte oder in den gleichen Gefäßen aufgewachsene Pflanzen ausgewählt oder sie wurden erst als Keimpflanzen getrennt angezogen und einige Tage vor der Behandlung in die Versuchsgefäße verpflanzt.

Je nach Wuchsform wurden die Testpflanzen bei einer Wuchshöhe von 3 bis 15 cm dann mit den in Wasser als Verteilungsmittel suspendierten oder emulgierten Wirkstoffen, die durch fein verteilende Düsen gespritzt wurden, behaldelt. Die Aufwandmengen für die Nachauflaufbehandlung variierten; sie betrugen 0,06 bis 3,0 kg Wirkstoff/ha.

Die Versuchsgefäße wurden im Gewächshaus aufgestellt, wobei für wärmeliebende Arten wärmere Bereiche (20 bis 35 °C) und für solche gemäßigter Klimate 10 bis 20 °C bevorzugt wurden. Die Versuchsperiode erstreckte sich über 2 bis 4 Wochen. Während dieser Zeit wurden die Pflanzen gepflegt und ihre Reaktion auf die einzelnen Behandlungen ausgewertet.

Die in den Gewächshausversuchen verwendeten Pflanzen setzten sich aus folgenden Arten zusammen:

| Lateinischer Name | Deutscher Name |
|---|---|
| Abutilon theophrasti | Chinesischer Hanf |
| Amaranthus spp. | Fuchsschwanzarten |
| Avena sativa | Hafer |
| Beta vulgaris | Rüben |
| Centaurea cyanus | Kornblume |
| Chenopodium album | Weißer Gänsefuß |
| Chrysantemum cor. | Kronenwucherblume |
| Galium aparine | Klettenlabkraut |
| Glycine max | Sojabohnen |
| Hordeum vulgare | Gerste |
| Mercurialis annua | Einjähriges Bingelkraut |
| Solanum nigrum | schwarzer Nachtschatten |
| Triticum aestivum | Weizen |
| Veronica spp. | Ehrenpreisarten |
| Viola tricolor | Gewöhnl. Stiefmütterchen |

Mit 3,0 kg Wirkstoff/ha im Nachauflaufverfahren eingesetzt, ließen sich mit den Wirkstoffen Nr. 69, 70 und 81 einkeim- und zweikeimblättrige Beispielpflanzen sehr gut bekämpfen.

Bei Nachauflaufanwendung von 0,25 kg Wirkstoff/ha wirkte der Wirkstoff Nr. 2 herbizid gegen eine Reihe von zweikeimblättrigen unerwünschten Pflanzen, wobei die Sojabohne nur geringfügige Schäden erlitt. Bei Nachauflaufapplikation der Wirkstoffe Nr..59 und 82 gelang die Bekämpfung wirtschaftlich bedeutender Schadpflanzen schon mit 0,06 kg Wirkstoff/ha. Die Kulturpflanze Soja wurde nicht oder nur unwesentlich beeinträchtigt.

Der Wirkstoff Nr. 13 eignete sich beispielsweise zur Bekämpfung der zweikeimblättrigen Unkrautflora in Getreide bei Nachauflaufanwendung von 0,06 kg Wirkstoff/ha.

Zur Bekämpfung breitblättriger Pflanzen im Nachauflaufverfahren eignen sich die Wirkstoffe Nr. 1, 4, 6, 8, 11, 60, 69 und 80. Bei der Behandlung mit 0,125 kg/ha des Wirkstoffs Nr. 69 wird die Zuckerrübe als Kulturpflanze allenfalls unwesentlich beeinträchtigt.

Galium aparine als Beispielunkraut in Gramineenkulturen läßt sich im Nachauflaufverfahren mit niedrigen Aufwandmengen (0,06 kg/ha) der Wirkstoffe Nr. 6 und 60 sicher bekämpfen, weder Weizen noch Gerste erleiden dabei nennenswerte Schädigung.

## Patentansprüche

**1.** N-substituierte 3,4,5,6-Tetrahydrophthalimide der allgemeinen Formel Ic

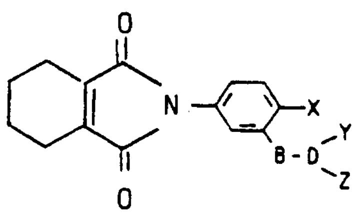

(Ic)

in der die Substituenten folgende Bedeutung haben:

B $-CH_2-$, $-CH_2-CHR^1-$, $-CH_2-CHR^1-CH_2-$, $-CH=$, $-CH=CR^1-$ oder $-CH=CR^1-CH=$, wobei der Rest $R^1$ für -H, -Cl, -Br oder $-CH_3$ steht,

D je nach der Endgruppe von B

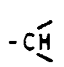 oder 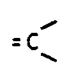 ,

X -H, -Cl oder -Br,

Y -H, $C_1$-$C_7$-Alkyl, -Cl, -Br, -CN, $CONH_2$ oder $-CO_2R^2$,

worin der Rest $R^2$ für H, $C_1$-$C_6$-Alkyl, $C_5$-$C_6$-Cycloalkyl, $C_1$-$C_4$-Alkoxy-$C_2$-$C_4$-alkyl, $C_1$-$C_4$-Alkylmercapto-$C_2$-$C_4$-alkyl, Propargyl, Benzyl, α-Phenylethyl, α-Phenylpropyl, durch F oder Cl bis zu dreifach substituiertes $C_2$-$C_4$-Alkyl oder durch $-CH_3$ oder -Cl substituiertes Allyl steht

Z

$-COOR^2$, $-CONR^3R^4$,

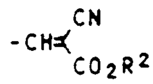

oder $-COR^2$,

worin die Reste $R^3$ und $R^4$ H und $C_1$-$C_4$-Alkyl bedeuten oder gemeinsam einen cycloaliphatischen 5- oder 6-Ring bilden, dessen Kohlenstoffkette durch ein Sauerstoffatom unterbrochen sein kann.

**2.** Anilinderivate der allgemeinen Formel Ib

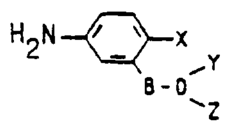

(Ib)

in der die Substituenten folgende Bedeutung haben:

B $-CH_2-$, $-CH_2-CHR^1-$, $-CH_2-CHR^1-CH_2-$, $-CH=$, $-CH=CR^1-$ oder $-CH=CR^1-CH=$, wobei der Rest $R^1$ für -H, -Cl, -Br oder $-CH_3$ steht.

D je nach der Endgruppe von B

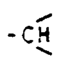 oder 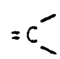 ,

X -H, -Cl oder -Br.

Y -H, $C_1$-$C_7$-Alkyl, -Cl, -Br, -CN, $CONH_2$ oder $-CO_2R^2$,

worin der Rest $R^2$ für H, $C_1$-$C_6$-Alkyl, $C_5$-$C_6$-Cycloalkyl, $C_1$-$C_4$-Alkoxy-$C_2$-$C_4$-alkyl, $C_1$-$C_4$-Alkylmer capto-$C_2$-$C_4$-alkyl, Propargyl, Benzyl, α-Phenylethyl, α-Phenylpropyl, durch F oder Cl bis zu dreifach substituiertes $C_2$-$C_4$-Alkyl oder durch $-CH_3$ oder -Cl substituiertes Allyl steht

Z

$-COOR^2$, $-CONR^3R^4$,

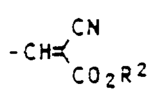

oder $-COR^2$,

worin die Reste $R^3$ und $R^4$ H und $C_1$-$C_4$-Alkyl bedeuten oder gemeinsam einen cycloaliphatischen 5- oder 6-Ring bilden, dessen Kohlenstoffkette durch ein Sauerstoffatom unterbrochen sein kann, ausgenommen 5-Amino-2-chlorzimtsäure.

**3.** Nitrobenzol-derivate der allgemeinen Formel Ia

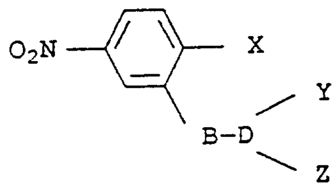

Ia

in der die Substituenten folgende Bedeutung haben:

B  $-CH_2-$, $-CH_2-CHR^1-$, $-CH_2-CHR^1-CH_2-$, $-CH=$, $-CH=CR^1-$ oder $-CH=CR^1-CH=$, wobei der Rest $R^1$ für Wasserstoff, Chlor, Brom oder Methyl steht,

D  je nach der Endgruppe von B

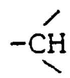

oder

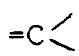 ,

X  Wasserstoff, Chlor oder Brom,

Y  Wasserstoff, $C_1$-$C_7$-Alkyl, Chlor, Brom, Cyano, $-CONH_2$ oder $-CO_2R^2$, worin der Rest $R^2$ für Wasserstoff, $C_1$-$C_6$-Alkyl, $C_5$-$C_6$-Cycloalkyl, $C_1$-$C_4$-Alkoxy-$C_2$-$C_4$-alkyl, $C_1$-$C_4$-Alkylmercapto-$C_2$-$C_4$-alkyl, Propargyl, Benzyl, α-Phenylethyl, α-Phenylpropyl, durch Fluor oder Chlor bis zu dreifach substituiertes $C_2$-$C_4$-Alkyl oder durch Methyl oder Chlor substituiertes Allyl steht;

Z  $-COOR^2$, $-CONR^3R^4$, $-CH=C(CN)-CO_2R^2$ oder $-COR^2$, worin die Reste $R^3$ und $R^4$ Wasserstoff und $C_1$-$C_4$-Alkyl bedeuten oder gemeinsam einen cycloaliphatischen 5- oder 6-gliedrigen Ring bilden, der durch ein Sauerstoffatom unterbrochen sein kann,

ausgenommen 2-Chlor-5-nitrozimtsäure, 3'-Nitro-6'-chlorbenzylidenacetessigsäuremethylester und 3'-Nitro-6'-chlorbenzylidenacetessigsäureethylester,
mit den Maßgaben, daß

- Y nicht Wasserstoff, $C_1$-$C_7$-Alkyl, -CN, -COOH oder $-CO_2C_2H_5$ bedeuten darf, wenn B für $-CH=$, D für

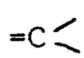 ,

X für Wasserstoff und Z für $-CO_2R^2$ mit $R^2$ = Wasserstoff oder $C_1$-$C_6$-Alkyl steht und

- Z nicht $-COCH_3$ oder $-COC_2H_5$ bedeuten darf, wenn B für $-CH=$, D für

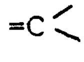 ,

X für Wasserstoff und Y für $-CO_2R^2$ mit $R^2$ = Wasserstoff, $C_1$-$C_6$-Alkyl, $C_5$-$C_6$-Cycloalkyl oder Propargyl steht.

**4.** Verfahren zur Herstellung von Nitrobenzolderivaten der Formel Ia gemäß Anspruch 3, <u>dadurch gekennzeichnet,</u> daß man die entsprechenden Nitrobenzaldehyde (IIa) Nitrophenylacetaldehyde (IIa') bzw. Nitrozimtaldehyde (IIa'')

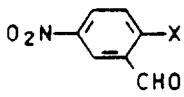 (IIa), 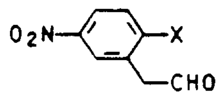 (IIa'), 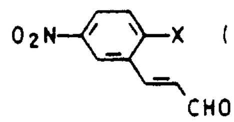 (IIa'')

in an sich bekannter Weise unter dehydratisierenden Bedingungen mit einer Verbindung (IIb)

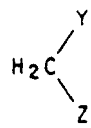 (IIb)

kondensiert und die hierbei erhältlichen Verbindungen Ia mit ungesättigter Seitenkette gewünschtenfalls hydriert oder für den Fall $R^1$ = Halogen nach Halogenanlagerung dehydrohalogeniert oder daß man die entsprechenden halogenfreien Ausgangsverbindungen (X = H) den gleichen Reaktionsbedingungen unterwirft und das Halogen in an sich bekannter Weise anschließend in das Molekül einführt.

**5.** Verfahren zur Herstellung von Anilinderivaten der Formel Ib gemäß Anspruch 2, dadurch gekennzeichnet, daß man die Nitroderivate Ia gemäß Anspruch 3 in an sich bekannter Weise zu den Anilinderivaten reduziert.

**6.** Verfahren nach Anspruch 5, dadurch gekennzeichnet, daß man als Reduktionsmittel Eisenpulver verwendet.

**7.** Verfahren nach Anspruch 5, dadurch gekennzeichnet , daß man als Reduktionsmittel Wasserstoff in Gegenwart eines Hydrierkatalysators verwendet.

**8.** Verfahren zur Herstellung der N-substituierten 3,4,5,6-Tetrahydrophthalimide der Formel Ic gemäß Anspruch 1, dadurch gekennzeichnet, daß man 3,4,5,6-Tetrahydrophthalsäureanhydrid in an sich bekannter Weise mit einem Anilinderivat der Formel Ib gemäß Anspruch 2 umsetzt.

**9.** Verwendung der N-substituierten 3,4,5,6-Tetrahydrophthalimide der Formel Ic gemäß Anspruch 1 als Herbizide.

**10.** Herbizid wirkende Mittel, enthaltend neben Zusatzstoffen N-substituierte 3,4,5,6-Tetrahydrophthalimide der Formel Ic gemaß Anspruch 1.

## Claims

**1.** An N-substituted 3,4,5,6-tetrahydrophthalimide of the formula Ic

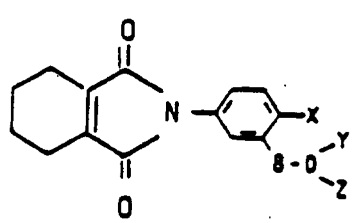 (Ic)

where
B is $-CH_2-$, $-CH_2-CHR^1-$, $-CH_2-CHR^1-CH_2-$, $-CH=$, $-CH=CR^1-$ or $-CH=CR^1-CH=$, $R^1$ being -H, -Cl, -Br or $-CH_3$, D is

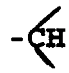

or

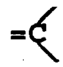 ,

depending on the terminal group B, X is -H, -Cl or -Br, Y is -H, $C_1$-$C_7$-alkyl, -Cl, -Br, -CN, $CONH_2$ or -$CO_2R^2$, where $R^2$ is H, $C_1$-$C_6$-alkyl, $C_5$- or $C_6$-cycloalkyl, $C_1$-$C_4$-alkoxy-$C_2$-$C_4$-alkyl, $C_1$-$C_4$-alkylmercapto-$C_2$-$C_4$-alkyl, propargyl, benzyl, $\alpha$-phenylethyl, $\alpha$-phenylpropyl, $C_2$-$C_4$-alkyl which is monosubstituted, disubstituted or trisubstituted by F or Cl, or -$CH_3$-substituted or -Cl- substituted allyl, and Z is Z -$COOR^2$, -$CONR^3R^4$,

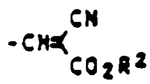

or -$COR^2$,
where $R^3$ and $R^4$ are each H or $C_1$-$C_4$-alkyl or together form a 5-membered or 6-membered cycloaliphatic ring whose carbon chain may be interrupted by an oxygen atom.

**2.** An aniline derivative of the formula Ib

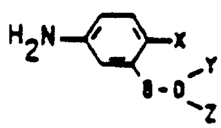

(Ib)

where
B is -$CH_2$-, -$CH_2$-$CHR^1$-, -$CH_2$-$CHR^1$-$CH_2$-, -CH =, -CH = $CR^1$- or -CH = $CR^1$-CH =, $R^1$ being -H, -Cl, -Br or -$CH_3$, D is

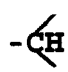

or

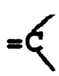 ,

depending on the terminal group B, X is -H, -Cl or -Br, Y is -H, $C_1$-$C_7$-alkyl, -Cl, -Br, -CN, -$CONH_2$ or -$CO_2R^2$, where $R^2$ is H, $C_1$-$C_6$-alkyl, $C_5$- or $C_6$-cycloalkyl, $C_1$-$C_4$-alkoxy-$C_2$-$C_4$-alkyl, $C_1$-$C_4$-alkylmercapto-$C_2$-$C_4$-alkyl, propargyl, benzyl, $\alpha$-phenylethyl, $\alpha$-phenylpropyl, $C_2$-$C_4$-alkyl which is monosubstituted, disubstituted or trisubstituted by F or Cl, or -$CH_3$-substituted or -Cl- substituted allyl, and Z is Z -$COOR^2$, -$CONR^3R^4$,

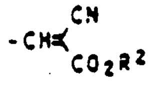

or -$COR^2$,
where $R^3$ and $R^4$ are each H or $C_1$-$C_4$-alkyl or together form a 5-membered or 6-membered cycloaliphatic ring whose carbon chain may be interrupted by an oxygen atom, with the exception of 5-amino-2-chlorocinnamic acid.

**3.** A nitrobenzene derivative of the formula Ia

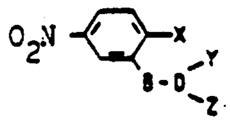

(Ia)

where

B is $-CH_2-$, $-CH_2-CHR^1-$, $-CH_2-CHR^1-CH_2-$, $-CH=$, $-CH=CR^1-$ or $-CH=CR^1-CH=$, $R^1$ being hydrogen, chlorine, bromine or methyl, D is

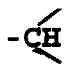

or

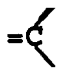 ,

depending on the terminal group of B, X is hydrogen, chlorine or bromine, Y is hydrogen, $C_1$-$C_7$-alkyl, chlorine, bromine, cyano, $-CONH_2$, or $-CO_2R^2$, where $R^2$ is hydrogen, $C_1$-$C_6$-alkyl, $C_5$- or $C_6$-cycloalkyl, $C_1$-$C_4$-alkoxy-$C_2$-$C_4$-alkyl, $C_1$-$C_4$-alkylmercapto-$C_2$-$C_4$-alkyl, propargyl, benzyl, α-phenylethyl, α-phenylpropyl, $C_2$-$C_4$-alkyl which is monosubstituted, disubstituted or trisubstituted by fluorine or chlorine, or methyl- or chlorine- substituted allyl, and Z is $-COOR^2$, $-CONR^3R^4$, $-CH=C(CN)-CO_2R^2$ or $-COR^2$ where $R^3$ and $R^4$ are each hydrogen or $C_1$-$C_4$-alkyl or together form a 5-membered or 6-membered cycloaliphatic ring which may be interrupted by an oxygen atom, with the exception of 2-chloro-5-nitrocinnamic acid, methyl 3'-nitro-6'-chlorobenzylideneacetoacetate and ethyl 3'-nitro-6'-chlorobenzylideneacetoacetate, with the provisos that Y may not be hydrogen, $C_1$-$C_7$-alkyl, -CN, -COOH or $-CO_2C_2H_5$ if B is $-CH=$, D is

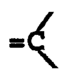 ,

X is hydrogen and Z is $-CO_2R^2$ where $R^2$ is hydrogen or $C_1$-$C_6$-alkyl, and Z may not be $-COCH_3$ or $-COC_2H_5$ if B is $-CH=$, D is

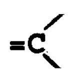 ,

X is hydrogen and Y is $-CO_2R^2$ where $R^2$ is hydrogen, $C_1$-$C_6$-alkyl, $C_5$- or $C_6$- cycloalkyl or propargyl.

**4.** A process for the preparation of a nitrobenzene derivative of the formula Ia as claimed in claim 3, wherein the corresponding nitrobenzaldehyde (IIa), nitrophenylacetaldehyde (IIa') or nitrocinnamaldehyde (IIa'')

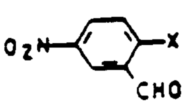 (IIa), 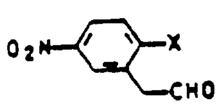 (IIa'), 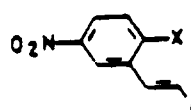 (IIa'') ,

is condensed with a compound (IIb)

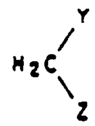

(IIb)

in a conventional manner under dehydrating conditions, and the resulting compound Ia having an unsaturated side chain is, if desired, hydrogenated, or, where $R^1$ is halogen, after halogen addition, is dehydrohalogenated, or the corresponding halogen-free starting compound (X = H) is subjected to the same reaction conditions and the halogen is then introduced into the molecule in a conventional manner.

**5.** A process for the preparation of an aniline derivative of the formula Ib as claimed in claim 2, wherein the nitro derivative Ia as claimed in claim 3 is reduced to the aniline derivative in a conventional manner.

**6.** A process as claimed in claim 5, wherein iron powder is used as the reducing agent.

**7.** A process as claimed in claim 5, wherein hydrogen in the presence of a hydrogenation catalyst is used as the reducing agent.

**8.** A process for the preparation of an N-substituted 3,4,5,6-tetrahydrophthalimide of the formula Ic as claimed in claim 1, wherein 3,4,5,6-tetrahydrophthalic anhydride is reacted with an aniline derivative of the formula Ib as claimed in claim 2, in a conventional manner.

**9.** Use of an N-substituted 3,4,5,6-tetrahydrophthalimide of the formula Ic as claimed in claim 1 as a herbicide.

**10.** A herbicide containing an N-substituted 3,4,5,6-tetrahydrophthalimide of the formula Ic as claimed in claim 1 in addition to additives.

**Revendications**

**1.** Tétrahydro-3,4,5,6-phtalimides substitués sur N, de la formule générale $I_c$

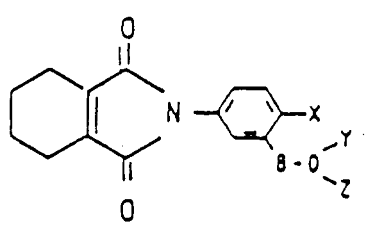

(Ic)

dans laquelle les substituants ont les significations suivantes :

B -$CH_2$-, -$CH_2$-$CHR^1$-$CH_2$-, -CH =, -CH = $CR^1$ ou -CH = $CR^1$-CH =, le reste $R^1$ étant mis pour -H, -Cl, -Br ou -$CH_3$

D, selon le groupe d'extrémité de B,

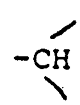

ou

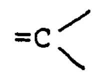

X, -H, -Cl ou -Br

Y, -H, alkyle en $C_1$-$C_7$, Cl, -Br, -CN, $CONH_2$ ou -$CO_2R^2$, le reste $R^2$ étant mis pour H, alkyle en $C_1$-$C_6$, cycloalkyle en $C_5$-$C_6$, alcoxy en $C_1$-$C_4$-alkyle en $C_2$-$C_4$, alkyl en $C_1$-$C_4$ mercapto-alkyle en $C_2$-$C_4$, propargyle, benzyle, $\alpha$-phényléthyle, $\alpha$-phénylpropyle, alkyle en $C_2$-$C_4$ substitué jusqu'à trois fois par F ou Cl, ou allyle substitué par -$CH_3$ ou -Cl

Z, -$COOR^2$, -$CONR^3R^4$,

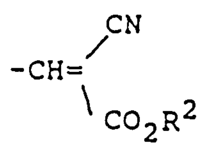

ou-$COR^2$ où les restes $R^3$ et $R^4$ représentent H et alkyle en $C_1$-$C_4$ ou forment ensemble un noyau cycloalyphatique à 5 à 6 chaînons, dont la chaîne de carbone peut être interrompue par un atome d'oxygène.

**2.** Dérivés d'aniline de la formule générale $I_b$

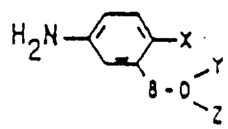

(Ib)

dans laquelle les substituants ont les significations suivantes

B -$CH_2$-, -$CH_2$-$CHR^1$, -$CH_2$-$CHR^1$-$CH_2$-, -CH=, -CH=$CR^1$ ou -CH=$CR^1$-CH=, le reste $R^1$ étant mis pour -H, -Cl, -Br ou -$CH_3$

D, selon le groupe d'extrémité de B,

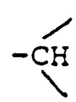

ou

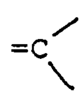

X, -H, -Cl ou -Br

Y, -H, alkyle en $C_1$-$C_7$, -Cl, -Br, -CN, $CONH_2$ ou -$CO_2R^2$, le reste $R^2$ étant mis pour H, alkyle en $C_1$-$C_6$, cycloalkyle en $C_5$-$C_6$, alcoxy en $C_1$-$C_4$-alkyle en $C_2$-$C_4$, alkyl en $C_1$-$C_4$ mercapto-alkyle en $C_2$-$C_4$, propargyle, benzyle, $\alpha$-phényléthyle, $\alpha$-phénylpropyle, alkyle en $C_2$-$C_4$ substitué jusqu'à trois fois par F ou Cl, ou allyle substitué par -$CH_3$ ou -Cl

Z, -$COOR^2$, -$CONR^3R^4$,

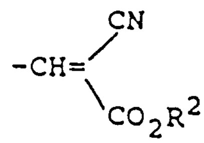

ou-$COR^2$
où les restes $R^3$ et $R^4$ représentent H et alkyle en $C_1$-$C_4$ ou forment ensemble un noyau cycloalyphatique à 5 à 6 chaînons, dont la chaîne de carbone peut être interrompue par un atome d'oxygène à l'exception de l'acide 5-amino-2-chlorocinnamique.

**3.** Dérivés de nitrobenzène de la formule générale $I_a$

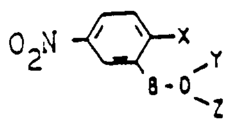 (Ia)

dans laquelle les substituants ont les significations suivantes :

B  $-CH_2-$, $-CH_2-CHR^1$, $-CH_2-CHR^1-CH_2-$, $-CH=$, $-CH=CR^1$ ou $-CH=CR^1-CH=$, le reste $R^1$ étant mis pour -H, -Cl, -Br ou $-CH_3$

D,  selon le groupe d'extrémité de B,

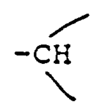

ou

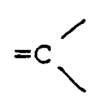

X,  -H, -Cl ou -Br

Y,  -H, alkyle en $C_1$-$C_7$, -Cl, -Br, -CN, $CONH_2$ ou $-CO_2R^2$, le reste $R^2$ étant mis pour H, alkyle en $C_1$-$C_6$, cycloalkyle en $C_5$-$C_6$, alcoxy en $C_1$-$C_4$-alkyle en $C_2$-$C_4$, alkyl en $C_1$-$C_4$ mercapto-alkyle en $C_2$(-$C_4$, propargyle, benzyle, $\alpha$-phényléthyle, $\alpha$-phénylpropyle, alkyle en $C_2$-$C_4$ substitué jusqu'à trois fois par F ou Cl, ou allyle substitué par $-CH_3$ ou -Cl

Z,  $-COOR^2$, $-CONR^3R^4$,

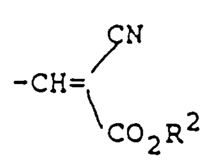

ou-$COR^2$
où les restes $R^3$ et $R^4$ représentent H et alkyle en $C_1$-$C_4$ ou forment ensemble un noyau cycloalyphatique à 5 à 6 chaînons, dont la chaîne de carbone peut être interrompue par un atome d'oxygène, exceptés acide 2-chloro-5-nitrocinnamique, 3'-nitro-6'-chloro-benzyliden-acétylacétate de méthyle et 3'-nitro-6'-chloro-benzyliden-acétylacétate d'éthyle
sous réserve que

- Y ne doit pas signifier hydrogène, alkyle en $C_1$-$C_7$, -CN, -COOH ou $-CO_2C_2H_5$, quand B est mis pour -CH, D pour

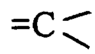 ,

X pour hydrogène et Z pour $-CO_2R^2$ avec $R^2$ = hydrogène ou alkyle en $C_1$-$C_6$, et

- Z ne doit pas représenter $-COCH_3$ ou $-COC_2H_5$ quand B est mis pour -CH=, D pour

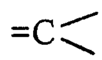 ,

X pour hydrogène et Y pour $-CO_2R^2$ avec $R^2$ = hydrogène, alkyle en $C_1$-$C_6$, cycloalkyle en $C_5$-$C_6$ ou propargyle.

**4.** Procédé de préparation de dérivés de nitrobenzène de la formule $I_a$ selon la revendication 3, caractérisé par le fait qu'on condense les nitrobenzaldéhyde (IIa), nitrophénylacétaldéhyde (IIa) et nitrocinnamaldéhyde ($II_a$'')

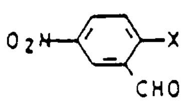 (IIa), 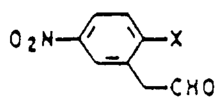 (IIa'), 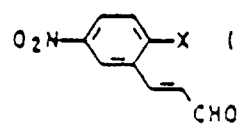 (IIa'')

de manière connue en soi, dans des conditions déshydratantes, avec un composé ($II_b$)

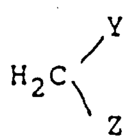 ($II_b$)

et, si on le souhaite, on hydrogène les composés $I_a$ ainsi obtenus avec une chaîne latérale insaturée ou bien, dans le cas où $R_1$ = halogène on déhydrohalogénise après fixation de l'halogène, ou encore on soumet les composés de départ (X = H) sans halogène correspondants aux mêmes conditions de réaction et on introduit de manière connue en soi l'halogène ensuite dans la molécule.

**5.** Procédé de préparation de dérivés d'aniline de la formule $I_b$ selon la revendication 2, caractérisé par le fait qu'on réduit, de manière connue en soi, les dérivés nitro $I_a$ selon la revendication 3 en les dérivés d'aniline.

**6.** Procédé selon la revendication 5, caractérisé par le fait qu'on utilise, comme agent de réduction, de la poudre de fer.

**7.** Procédé selon la revendication 5, caractérisé par le fait qu'on utilise, comme agent de réduction, de l'hydrogène en présence d'un catalyseur d'hydrogénation.

**8.** Procédé de préparation de tétrahydro-3,4,5,6-phtalimides substitués sur N de la formule générale $I_c$, selon la revendication 1, caractérisé par le fait qu'on fait réagir de l'anhydide d'acide tétrahydro-3,3,5,6-phtalique, de manière connue en soi, avec un dérivé d'aniline de la formule $I_b$ selon la revendication 2.

**9.** Utilisation du tétrahydro-3,4,5,6-phtalimide substitué sur N de la formule générale $I_c$ selon la revendication 1, comme herbicide.

**10.** Agent à action herbicide, contenant, outre les additifs, du tétrahydro-3,4,5,6-phtalimide substitué sur N de la formule générale $I_c$, selon la revendication 1.